# EUROPEAN PATENT APPLICATION

(11) **EP 4 763 051 A1**
(43) Date of publication of application: **24.06.2026**
(21) Application number: 24220610.0
(22) Date of filing: 17.12.2024
(51) Int. Cl.: A61B 5/00, A61B 5/25

(54) **AN ELECTRODE AND A WEARING DEVICE**

(71) Applicant: GN Hearing A/S, 2750 Ballerup (DK)
(72) Inventor: KRISTENSEN, Peter Rechnagel, 2750 Ballerup (DK); SCHWARTZ, Michael Sødal, 2750 Ballerup (DK); ANGELO, Casper Stein, 2750 Ballerup (DK); NIELSEN, Martin Egholm, 2750 Ballerup (DK)
(74) Representative: Zacco Denmark A/S

(57) **Abstract**

Disclosed is an electrode (100) configured to be arranged on a user's skin for recording electrical activity. The electrode (100) comprises a first magnetic material (110) extending along a first plane (XY). The first magnetic material (110) comprising a first surface (112) being configured to be arranged on the user's skin. The electrode (100) comprises a magnet (120) arranged in a distance (D) away from the first magnetic material (110) such that the first magnetic material (110) magnetically interacts with the magnet (120). The electrode (100) comprises an attaching member (130) arranged in contact with the first magnetic material (110) and with the magnet (120) to maintain the distance (D) between the first magnetic material (110) and the magnet (120).

## Description

### FIELD

The present invention relates to an electrode and a wearing device comprising said electrode. More specifically, the disclosure relates to an electrode configured to be arranged on a user's skin for recording electrical activity and a wearing device comprising one or more of said electrode.

### BACKGROUND

The measurement of electrical activity from users, such as bioelectrical signals from the skin, plays a critical role in various fields, including medical diagnostics, fitness monitoring, and human-computer interaction. Electrodes are commonly used as interfaces to detect and record these signals, such as electrocardiograms (ECG), electroencephalograms (EEG), and electromyograms (EMG). Traditional electrodes often rely on gel-based materials to enhance conductivity between the skin and the electrode surface. Although using gel-based materials enhances the conductivity, it may cause skin irritation, limited reusability, and drying over time, which can degrade signal quality. Dry electrodes do not require gels but often face challenges in achieving consistent contact quality, particularly under motion or in non-optimal environmental conditions.

Thus, there is a need for an improved electrode which addresses or at least mitigates the abovementioned challenges.

### SUMMARY

It is an object of the invention to provide an improved electrode with improved contact with a user's skin without discomfort for the user. It is another object of the invention to provide an improved electrode suitable for various users with various skin types and preferences. It is yet another object of the invention to provide an improved electrode which may be replaced by the user in a simple and user-friendly manner.

According to a first aspect of the invention, disclosed is an electrode. The electrode is configured to be arranged on a user's skin for recording electrical activity. The electrode comprises a first magnetic material. The first magnetic material extends along a first plane. The first magnetic material comprises a first surface. The first surface is configured to be arranged on the user's skin. The electrode further comprises a magnet arranged in a distance away from the first magnetic material such that the first magnetic material magnetically interacts with the magnet. The electrode further comprises an attaching member arranged in contact with the first magnetic material and with the magnet to maintain the distance between the first magnetic material and the magnet.

It is an advantage that the electrode is configured to be arranged on a user's skin for recording electrical activity, as the electrode allows for converting the bioelectrical signals, generated by the user's body, into electrical signals which may be measured and analyzed by an external system. The electrode may be arranged on various parts of the user's body depending on the desired electrical activity to be recorded. For instance, in Electromyography (EMG), the electrode may be arranged on the user's chest to record the electrical activity produced by skeletal muscles of the chest. In Electrocardiography, the electrode may be arranged on the user's chest to record the electrical activity produced by user's heart. In Electroencephalography (EEG), the electrode may be arranged on the user's head to record the electrical activity produced by user's brain.

The electrode comprises the first magnetic material. The first magnetic material extends along the first plane. The first magnetic material comprises the first surface. The first surface of the first magnetic material may extend along the first plane. The first surface is configured to be arranged on the user's skin. Thereby, the first surface of the first magnetic material is configured to have contact with the user's skin to record the desired electrical activity of the user's body. The first magnetic material may have other surfaces. It is an advantage that the electrode comprises the first magnetic material, as it allows for the first magnetic material to be designed in a simple and user-friendly manner. It is an advantage that the first magnetic material extends along the first plane, as an area of the first magnetic material extending along the first plane may be designed in a flexible and desired manner. For instance, the area of the first surface of the first magnetic material extending along the first plane may be designed in a flexible and desired manner.

The electrode further comprises the magnet. The magnet is arranged in the distance away from the first magnetic material. It is an advantage that the magnet is arranged away from the user's skin, as it prevents, or at least mitigates, any skin irritation, allergic reactions, or any interaction of the magnet with other medical devices that that user may use. The distance between the first magnetic material and the magnet may be understood as a minimum distance between the first magnet material and the magnet.

The first magnetic material magnetically interacts with the magnet. The first magnetic material may be attracted to the magnet. The first magnetic material may comprise any material suitable for magnetically interact with the magnet. The magnet is advantageous as it allows for maintaining a position/location of the first magnetic material on the user's skin in a stable and robust manner.

The first magnetic material may comprise any material suitable for becoming magnetized and/or attracted to the magnet. The first magnetic material may be any magnetic material such as a ferromagnetic material or alloy or other material with similar magnetic properties such as relative permeability. The first magnetic material may have a relative permeability (µ_{/}µ₀) of at least 100. The magnet may be any type of magnet capable of magnetically interacting with the first magnetic material such as a permanent magnet or an electromagnet. The magnet may comprise any magnet such as a ferrite magnet.

The electrode further comprises the attaching member. The attaching member is arranged in contact with the first magnetic material and with the magnet to maintain the distance between the first magnetic material and the magnet. Thereby, the attaching member allows for maintain the distance between the first magnetic material and the magnet. The attaching member also allows for maintaining the position/location of the first magnetic material relative to the magnet. Thus, the attaching member facilitates the magnetic interaction between the magnet and the first magnetic material. The attaching member further prevents, or at least mitigates, a risk of the electrode dislodging during removal of the electrode from the user's skin. The attaching member may be any components configured for maintaining the distance between the first magnetic material and the magnet. An example of the attaching member may be a rod, a cylinder, a block, a housing, a glue, or anything suitable of maintaining the distance between the first magnetic material and the magnet. The magnet may comprise a cavity for receiving the attaching member thereto. In the case that the attaching member is a rod, the magnet may have a ring shape to receive a portion of the attaching member thereto.

In overall, the electrode is advantageous in that it allows for maintaining the position/location of the first magnetic material on the user's skin in a simple, stable and robust manner. Thereby, it allows for an improved contact with a user's skin without discomfort for the user. The electrode is further advantageous in that it allows for designing properties of the electrode in a flexible and user-friendly manner such as modifying a size of the electrode or properties e.g. surface roughness of the first magnetic material manner. The electrode is further advantageous in that it allows for a durable electrode which may be cleaned in a user-friendly manner. This in turn allows for a cost-efficient electrode with improved hygiene.

The electrode may have any shape or dimension along the first plane. The dimension of the electrode along the first plane may be defined by a dimension of the first magnetic material along the first plane. The first magnetic material may have a round shape, such as a circular shape or an oval shape. In such case, the dimension of the first magnetic material along the first plane may correspond to a diameter of the first magnetic material.

In an embodiment, a hearing device is configured to be worn by a user. The hearing device may be arranged at the user's ear, on the user's ear, over the user's ear, in the user's ear, in the user's ear canal, behind the user's ear and/or in the user's concha, i.e., the hearing device is configured to be worn in, on, over and/or at the user's ear. The user may wear two hearing devices, one hearing device at each ear. The two hearing devices may be connected, such as wirelessly connected and/or connected by wires, such as a binaural hearing aid system.

The hearing device may be a hearable such as a headset, headphone, earphone, earbud, hearing aid, a personal sound amplification product (PSAP), an over-the-counter (OTC) hearing device, a hearing protection device, a one-size-fits-all hearing device, a custom hearing device or another head-wearable hearing device. Hearing devices can include both prescription devices and non-prescription devices.

The hearing device may be embodied in various housing styles or form factors. Some of these form factors are Behind-the-Ear (BTE) hearing device, Receiver-in-Canal (RIC) hearing device, Receiver-in-Ear (RIE) hearing device or Microphone-and-Receiver-in-Ear (MaRIE) hearing device. These devices may comprise a BTE unit configured to be worn behind the ear of the user and an in the ear (ITE) unit configured to be inserted partly or fully into the user's ear canal. Generally, the BTE unit may comprise at least one input transducer, a power source and a processing unit. The term BTE hearing device refers to a hearing device where the receiver, i.e. the output transducer, is comprised in the BTE unit and sound is guided to the ITE unit via a sound tube connecting the BTE and ITE units, whereas the terms RIE, RIC and MaRIE hearing devices refer to hearing devices where the receiver may be comprised in the ITE unit, which is coupled to the BTE unit via a connector cable or wire configured for transferring electric signals between the BTE and ITE units.

Some of these form factors are In-the-Ear (ITE) hearing device, Completely-in-Canal (CIC) hearing device or Invisible-in-Canal (IIC) hearing device. These hearing devices may comprise an ITE unit, wherein the ITE unit may comprise at least one input transducer, a power source, a processing unit and an output transducer. These form factors may be custom devices, meaning that the ITE unit may comprise a housing having a shell made from a hard material, such as a hard polymer or metal, or a soft material such as a rubber-like polymer, molded to have an outer shape conforming to the shape of the specific user's ear canal.

Some of these form factors are earbuds, on the ear headphones or over the ear headphones. The person skilled in the art is well aware of different kinds of hearing devices and of different options for arranging the hearing device in, on, over and/or at the ear of the hearing device wearer. The hearing device (or pair of hearing devices) may be custom fitted, standard fitted, open fitted and/or occlusive fitted.

In an embodiment, the hearing device may comprise one or more input transducers. The one or more input transducers may comprise one or more microphones. The one or more input transducers may comprise one or more vibration sensors configured for detecting bone vibration. The one or more input transducer(s) may be configured for converting an acoustic signal into a first electric input signal. The first electric input signal may be an analogue signal. The first electric input signal may be a digital signal. The one or more input transducer(s) may be coupled to one or more analogue-to-digital converter(s) configured for converting the analogue first input signal into a digital first input signal.

In an embodiment, the hearing device may comprise one or more antenna(s) configured for wireless communication. The one or more antenna(s) may comprise an electric antenna. The electric antenna may be configured for wireless communication at a first frequency. The first frequency may be above 800 MHz, preferably a wavelength between 900 MHz and 6 GHz. The first frequency may be 902 MHz to 928 MHz. The first frequency may be 2.4 to 2.5 GHz. The first frequency may be 5.725 GHz to 5.875 GHz. The one or more antenna(s) may comprise a magnetic antenna. The magnetic antenna may comprise a magnetic core. The magnetic antenna may comprise a coil. The coil may be coiled around the magnetic core. The magnetic antenna may be configured for wireless communication at a second frequency. The second frequency may be below 100 MHz. The second frequency may be between 9 MHz and 15 MHz.

In an embodiment, the hearing device may comprise one or more wireless communication unit(s). The one or more wireless communication unit(s) may comprise one or more wireless receiver(s), one or more wireless transmitter(s), one or more transmitter-receiver pair(s) and/or one or more transceiver(s). At least one of the one or more wireless communication unit(s) may be coupled to the one or more antenna(s). The wireless communication unit may be configured for converting a wireless signal received by at least one of the one or more antenna(s) into a second electric input signal. The hearing device may be configured for wired/wireless audio communication, e.g. enabling the user to listen to media, such as music or radio and/or enabling the user to perform phone calls.

In an embodiment, the wireless signal may originate from one or more external source(s) and/or external devices, such as spouse microphone device(s), wireless audio transmitter(s), smart computer(s) and/or distributed microphone array(s) associated with a wireless transmitter. The wireless input signal(s) may origin from another hearing device, e.g., as part of a binaural hearing system and/or from one or more accessory device(s), such as a smartphone and/or a smart watch.

In an embodiment, the hearing device may include a processing unit. The processing unit may be configured for processing the first and/or second electric input signal(s). The processing may comprise compensating for a hearing loss of the user, i.e., apply frequency dependent gain to input signals in accordance with the user's frequency dependent hearing impairment. The processing may comprise performing feedback cancelation, beamforming, tinnitus reduction/masking, noise reduction, noise cancellation, speech recognition, bass adjustment, treble adjustment and/or processing of user input. The processing unit may be a processor, an integrated circuit, an application, functional module, etc. The processing unit may be implemented in a signal-processing chip or a printed circuit board (PCB). The processing unit may be configured to provide a first electric output signal based on the processing of the first and/or second electric input signal(s). The processing unit may be configured to provide a second electric output signal. The second electric output signal may be based on the processing of the first and/or second electric input signal(s).

In an embodiment, the hearing device may comprise an output transducer. The output transducer may be coupled to the processing unit. The output transducer may be a receiver. It is noted that in this context, a receiver may be a loudspeaker, whereas a wireless receiver may be a device configured for processing a wireless signal. The receiver may be configured for converting the first electric output signal into an acoustic output signal. The output transducer may be coupled to the processing unit via the magnetic antenna. The output transducer may be comprised in an ITE unit or in an earpiece, e.g. Receiver-in-Ear (RIE) unit or Microphone-and-Receiver-in-Ear (MaRIE) unit, of the hearing device. One or more of the input transducer(s) may be comprised in an ITE unit or in an earpiece.

In an embodiment, the wireless communication unit may be configured for converting the second electric output signal into a wireless output signal. The wireless output signal may comprise synchronization data. The wireless communication unit may be configured for transmitting the wireless output signal via at least one of the one or more antennas.

In an embodiment, the hearing device may comprise a digital-to-analogue converter configured to convert the first electric output signal, the second electric output signal and/or the wireless output signal into an analogue signal.

In an embodiment, the hearing device may comprise a vent. A vent is a physical passageway such as a canal or tube primarily placed to offer pressure equalization across a housing placed in the ear such as an ITE hearing device, an ITE unit of a BTE hearing device, a CIC hearing device, a RIE hearing device, a RIC hearing device, a MaRIE hearing device or a dome tip/earmold. The vent may be a pressure vent with a small cross section area, which is preferably acoustically sealed. The vent may be an acoustic vent configured for occlusion cancellation. The vent may be an active vent enabling opening or closing of the vent during use of the hearing device. The active vent may comprise a valve.

In an embodiment, the hearing device may comprise a power source. The power source may comprise a battery providing a first voltage. The battery may be a rechargeable battery. The battery may be a replaceable battery. The power source may comprise a power management unit. The power management unit may be configured to convert the first voltage into a second voltage. The power source may comprise a charging coil. The charging coil may be provided by the magnetic antenna.

In an embodiment, the hearing device may comprise a memory, including volatile and nonvolatile forms of memory.

The first magnetic material may further comprise a second surface. The second surface may be arranged opposite to the first surface. The magnet may comprise a first surface. The first surface of the magnet may face the second surface of the first magnetic material. The distance may correspond to a distance between the first surface of the magnet and the second surface of the first magnetic material along a direction extending perpendicular to the first plane.

The first surface of the magnet may extend along the first plane. The magnet may have a second surface. The second surface of the magnet may be arranged opposite to the first surface of the magnet. There may be several different distances between the first surface of the magnet and the second surface of the first magnetic material. The distance may correspond to a minimum distance between the first surface of the magnet and the second surface of the first magnetic material along the direction. The electrode may have a height along the direction. The height of the electrode along the direction may be in the range of 0.3 mm to 1 mm.

The electrode further may further comprise a second magnetic material. The second magnetic material may extend along the first plane. The second magnetic material may comprise a first surface and a second surface. The second surface of the second magnetic material may be arranged opposite to the first surface. The first surface of the second magnetic material may be configured to be arranged on the user's skin. The second surface of the second magnetic material may be configured to magnetically interact with the magnet and be attached on the first surface of the first magnetic material.

It is an advantage that the second surface of the second magnetic material may be configured to magnetically interact with the magnet and may be attached on the first surface of the first magnetic material, as the second magnetic material may attach to the electrode. Thereby, the second magnetic material may not dislodge. It is advantageous that the user may arrange the second magnetic material on the first magnetic material in a simple and user-friendly manner, without needing any help from a professional. The second magnetic material may allow for an improved electrode, as the user may attach the second magnetic material on the first magnetic material when desired. The second magnetic material may be different from the first magnetic material. The first surface of the second magnetic material may have different properties from the first surface of the first magnetic material. For instance, the first surface of the second magnetic material may be rougher than the first surface of the first magnetic material. In such case and when the electrode needs to be arranged on a hairy area of the user's skin, the user may arrange the second magnetic material on the first magnetic material to improve the contact of the electrode with the user's skin. Thus, the second magnetic material may allow for an improved contact of the electrode with the user's skin i.e. to record the electrical activity to record desired electrical activity of the user. The electrode may comprise a plurality of the second magnetic materials such as two or three second magnetic materials. The second magnetic material may be any magnetic material such as a ferromagnetic material or alloy or other material with similar magnetic properties such as relative permeability.

The second magnetic material may have the same shape and size as the first magnetic material. The second magnetic material does not need to have the same shape or size as the first magnetic material. The second magnetic material may have a larger dimension than the first magnetic material. The second magnetic material may have a smaller dimension than the first magnetic material. The magnet may have the same shape and size as the first magnetic material. The magnet does not need to have the same shape or size as the first magnetic material. The magnet may have a larger dimension than the first magnetic material. The magnet may have a smaller dimension than the first magnetic material. The material of the second magnetic material may be the same as the first magnetic material. The material of the second magnetic material does not need to be the same as the material of the first magnetic material. The second magnetic material may have a relative permeability (µ_{/}µ₀) of at least 100.

The second magnetic material may comprise a first pattern at the first surface. The first pattern may comprise a protrusion and/or a depression.

It is an advantage, that the second magnetic material may comprise the first pattern at the first surface, in that the pattern may allow for providing an improved contact to the user's skin. The first pattern may be customized to the needs of the user. The first pattern may be configured to fit the shape of the skin of the users desired. The first pattern may provide an improved comfort to the user, as the pattern may prevent heating or sticking of the second magnetic material on the user's skin, as it may allow for heat or sweat from the user's skin to travel between the second magnetic material and the user's skin.

The first pattern may comprise the protrusion. The protrusion may comprise a dot, a line, or a shape. For instance, the protrusion may be a dimple protruding out of the first surface of the second magnetic material. It is an advantage that the first pattern may comprise the protrusion, as the protrusion may improve contact between the user's skin and the first surface of the second magnetic materials. For instance, in hairy areas of the user's skin e.g. the head of the user, the protrusion may improve contact to the user's skin.

The first pattern may comprise the depression. The depression may comprise a dot, a line, or a shape. For instance, the depression may be a shape e.g. a logo indented into the first surface the first surface of the second magnetic material. It is an advantage that the first pattern may comprise the depression, as the depression may improve comfort in bony areas of the user's body, such as a wrist of the user.

The first pattern may comprise a plurality of protrusions. The plurality of protrusion may comprise any of or any combination of dots, lines, or shapes protruding out of the first surface of the second magnetic material. Alternatively or in combination, the first pattern may comprise a plurality of depressions. The plurality of depressions may comprise any of or any combination of dots, lines, or shapes indented into the first surface the first surface of the second magnetic material. The plurality of protrusions and/or the plurality of depressions may be arranged in an order manner across the first surface of the second magnetic material. The plurality of protrusions and/or the plurality of depressions may be arranged randomly across the first surface of the second magnetic material.

The first magnetic material may comprise a second pattern at the first surface. The second pattern may comprise a protrusion and/or a depression.

It is an advantage, that the first magnetic material may comprise the second pattern at the first surface, in that the second pattern may allow for providing an improved contact to the user's skin. The second pattern may be customized to the needs of the user. The second pattern may be configured to fit the shape of the skin of the user, as desired. The second pattern may provide an improved comfort to the user, as the second pattern may prevent heating or sticking of the second magnetic material on the user's skin, as it may allow for heat or sweat from the user's skin to travel between the second magnetic material and the user's skin.

The second pattern may comprise the protrusion. The protrusion may comprise a dot, a line, or a shape. For instance, the protrusion may be a dimple protruding out of the first surface of the first magnetic material. It is an advantage that the second pattern may comprise the protrusion, as the protrusion may improve contact between the user's skin and the first surface of the first magnetic materials. For instance, in hairy areas of the user's skin e.g. the head of the user, the protrusion may improve contact to the user's skin.

The second pattern may comprise the depression. The depression may comprise a dot, a line, or a shape. For instance, the depression may be a shape e.g. a logo indented into the first surface the first surface of the first magnetic material. It is an advantage that the second pattern may comprise the depression, as the depression may improve comfort in bony areas of the user's body, such as a wrist of the user.

The second pattern may comprise a plurality of protrusions. The plurality of protrusion may comprise any of or any combination of dots, lines, or shapes protruding out of the first surface of the first magnetic material. Alternatively, or in combination, the second pattern may comprise a plurality of depressions. The plurality of depressions may comprise any of or any combination of dots, lines, or shapes indented into the first surface the first surface of the first magnetic material. The plurality of protrusions and/or the plurality of depressions may be arranged in an order manner across the first surface of the first magnetic material. The plurality of protrusions and/or the plurality of depressions may be arranged randomly across the first surface of the first magnetic material.

The first pattern and/or the second pattern may comprise any of or any combination of a shape, a line or a dot. Thereby, the shape, the line, or the dot may improve surface roughness of the respective magnetic material and thus contact to the user's skin. There may also provide information to the user e.g. indicating which side of the second magnetic material should be attached to the first surface of the first magnetic material.

The first surface of the first magnetic material may comprise an indentation. The second surface of the second magnetic material may comprise a corresponding protrusion. When the second surface of the second magnetic material is magnetically attached to the first surface of first magnetic material, the protrusion of the second surface of the second magnetic material may be received into the indentation of the first surface of the first magnetic material. The indentation of the first surface of the first magnetic material and the corresponding protrusion of the second surface of the second magnetic material may allow for the second magnetic material to attach on the first magnetic material in a more stable and aligned manner.

The more stable attachment may in turn reduce relative movement between the first magnetic material and the second magnetic material along the first plane. The indentation of the first surface of the first magnetic material and the corresponding protrusion of the second surface of the second magnetic material may notify the user that the second magnetic material is correctly arranged on the first magnetic material.

The first magnetic material and/or the second magnetic material may comprise a coating layer covering at least the first surface of the respective magnetic material.

It is an advantage that the first magnetic material and/or the second magnetic material may comprise the coating layer, as the coating layer may improve durability of the first surface of the respective magnetic material, e.g. the coating may protect the respective magnetic material against corrosion, oxidation, etc. The coating layer may completely cover the respective magnetic material i.e. the coating layer may cover all surfaces of the respective magnetic material. The coating layer may partially cover the respective magnetic material. For instance, the coating layer may cover the first surface of the respective magnetic material.

The coating layer may preferably comprise gold. The coating layer may comprise carbon e.g. a carbon-based material. The coating layer may be electrically conductive. Thus, it may facilitate recording the electrical activity from the user's skin or the second surface of an additional magnetic material, such as conducting electrical signals from the second surface of the second magnetic material to the first magnetic material. The coating layer may have a thickness in the range of 0.5 µm to 2.5 µm. The coating layer may be applied in a manner which per se is known in the field such as plating. For instance, the coating layer may be applied using electro-plating.

The first magnetic material and/or the second magnetic material may have a round shape such as a circular shape.

Thereby, the round shape may increase comfortability to the user, as the round shape does not have any corners. The first magnetic material and/or the second magnetic material may have the round shape along the first plane, such as a circular shape or an oval shape along the first plane.

A thickness of the first magnetic material and/or a thickness of the second magnetic material along the direction may be in the range of 0.2 mm to 0.6 mm.

The thickness of the first magnetic material and/or the thickness of the second magnetic material may allow for the electrode to be minimally noticeable on the user's skin, while still maintaining improved contact with the user's skin without discomfort for the user.

By the term thickness is hereby meant a dimension of the first magnetic material or a dimension of the second magnetic material in the direction perpendicular to the first plane.

A dimension of the first magnetic material and/or a dimension of the second magnetic material along the first plane may be in the range of 5 mm to 20 mm.

Thereby, the dimension of first magnetic material or the dimension of the second magnetic material, in the range of 5 mm to 20 mm, may provide the improved contact with the user's skin while still being minimally noticeable on the user's skin.

The attaching member may comprise a spring.

The spring is advantageous in that it may facilitate maintaining the distance between the first magnetic material and the magnet. The spring may be any type of spring, such as a disc spring, a star spring, a helical spring, a compression spring, or an extension spring. The attaching member may comprise a plurality of springs. The plurality of springs may comprise any of or any combination of the disc spring, the star spring, the helical spring, the compression spring, or the extension spring. The attaching member may be any other attaching member such as a binding agent, such as glue. The attaching member may be any conventional and commercially available attaching member.

According to a second aspect of the invention, disclosed is a wearing device configured to be worn by a user. The wearing device comprises an electrode according to the first aspect of the invention. The first magnetic material of the electrode is arranged on a first surface of the wearing device. The first surface of the wearing device is configured to be arranged on a user's skin. The magnet and the attaching member of the electrode are arranged in the wearing device.

This aspect may generally present the same or similar advantages as defined above in relation to the first aspect of the invention.

It is an advantage that the wearing device comprises the electrode, as the user may wear the wearing device in a simple, user-friendly and convenient manner while the electrode records the electrical activity from the user throughout a day. Thereby, the wearing device allows for the user to perform other activities while the electrode records the electrical activity from the user. It is an advantage, that the first magnetic material of the electrode is arranged on the first surface of the wearing device, in that it allows the electrode to have access to record electrical activity. It is an advantage, that the magnet and the attaching member of the electrode are arranged in the wearing device, as it allows for a user-friendly wearing device since the magnet and attaching member of the electrode are not arranged on the surface e.g. the first surface of the wearing device. The magnet and the attaching member of the electrode may be arranged inside the wearing device. For instance, the magnet and the attaching member of the electrode may be arranged inside a housing, the housing being arranged inside the wearing device. A wearing device may be any wearing device configured to be worn by the user. A wearing device may be a hat, a band such as a wrist band, a chest band, a headband, a leg band, or any other wearing device.

The wearing device may be a headset. The headset may comprise at least an earphone and a headband. The first magnetic material of the electrode may be arranged on a first surface of the earphone. The first surface of the earphone may be configured to be arranged on a user's skin when in use. The magnet and the attaching member of the electrode may be arranged in the earphone.

Thereby, the headset may record electrical activity of the user when the user is wearing the headset and while the user is performing other activities such as listening to music. It is an advantage, that the first magnetic material of the electrode is arranged on the first surface of the earphone, in that the earphone allows for, or at least facilitates, contacts to the user's skin for the electrode. It is an advantage, that the magnet and the attaching member of the electrode are arranged in the earphone, in that the magnet and attaching member of the electrode may be protected by the earphone and carried comfortably by the user. The wearing device may be a headphone, a hearing device, a headset, an earphone, a hearing aid, a personal sound amplification product (PSAP), an over-the-counter (OTC) hearing device, a hearing protection device, a one-size-fits-all hearing device, a custom hearing device or another head-wearable hearing device.

The headset may comprise a plurality of electrodes arranged across the first surface of the earphone such as four electrodes.

It an advantage, that the headset comprises a plurality of electrodes arranged across the first surface of the earphone such as four electrodes, in that a plurality of electrodes may improve recording electrical activity. Thereby, the may allow for obtaining an improved electrical activity e.g. by correlating recorded electrical activity obtained from different electrodes. The plurality of electrodes may be similar to one another. The plurality of electrodes does not need to be similar to one another. For instance, they may comprise a different type of first magnetic material, or a different type of second magnetic material, etc. The plurality of the electrodes may also allow for recording different types of electrical activity.

The present invention relates to different aspects including the electrode and the wearing device described above and in the following, and corresponding electrode parts and wearing device parts, each yielding one or more of the benefits and advantages described in connection with the first mentioned aspect, and each having one or more embodiments corresponding to the embodiments described in connection with the first mentioned aspect and/or disclosed in the appended claims.

### BRIEF DESCRIPTION OF THE DRAWINGS

The above and other features and advantages will become readily apparent to those skilled in the art by the following detailed description of exemplary embodiments thereof with reference to the attached drawings, in which:
Fig. 1 schematically illustrates a side view of an exemplary electrode comprising a first magnetic material.
Fig. 2 schematically illustrates a side view of another exemplary electrode comprising a first magnetic material and a second magnetic material.
Fig. 3 schematically illustrates a perspective view of another exemplary electrode, comprising a first magnetic material and a second magnetic material.
Fig. 4a schematically illustrates a top view of an exemplary wearing device comprising two electrodes each comprising a first magnetic material.
Fig. 4b schematically illustrates a cross-sectional view of the wearing device shown in Fig. 4a.
Fig. 5a schematically illustrates a front view of an exemplary headset comprising a headband and two earphones.
Fig. 5b schematically illustrates an inside view of the right earphone shown in Fig. 5a comprising four electrodes.

### DETAILED DESCRIPTION

Various embodiments are described hereinafter with reference to the figures. Like reference numerals refer to like elements throughout. Like elements will, thus, not be described in detail with respect to the description of each figure. It should also be noted that the figures are only intended to facilitate the description of the embodiments. They are not intended as an exhaustive description of the claimed invention or as a limitation on the scope of the claimed invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

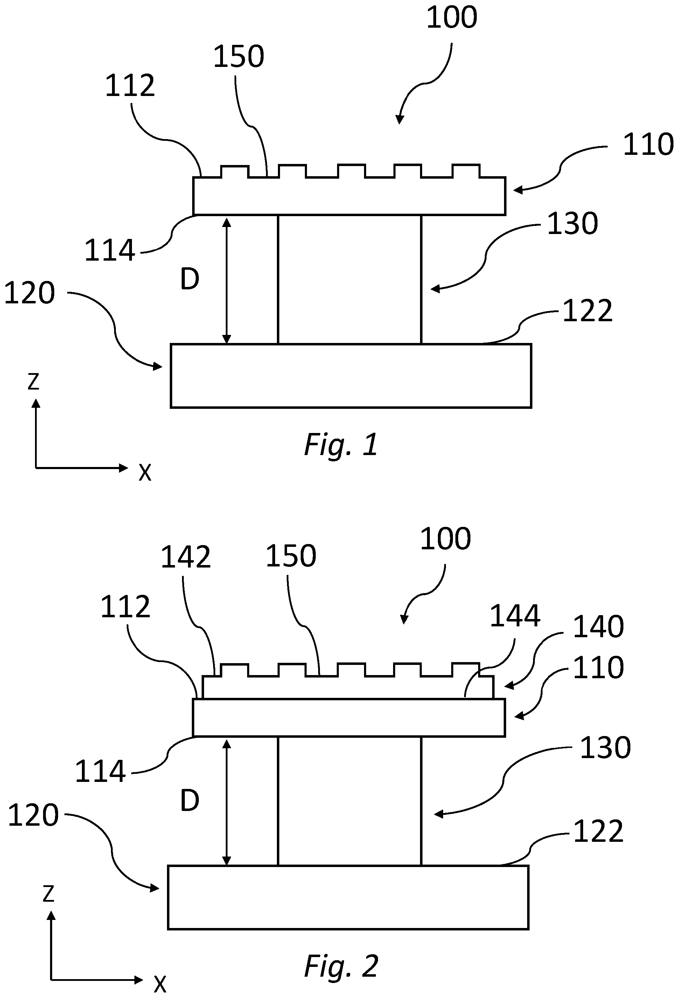

Fig. 1 schematically illustrates a side view of an exemplary electrode 100. The electrode 100 is configured to be arranged on a user's skin for recording electrical activity. The electrode 100 comprises a first magnetic material 110. The first magnetic material 110 extends along a first plane XY. The first magnetic material 110 comprises a first surface 112. The first surface 112 is configured to be arranged on the user's skin. The electrode 100 further comprises a magnet 120. The magnet 120 is arranged in a distance D away from the first magnetic material 110, such that the first magnetic material 110 magnetically interacts with the magnet 120. The electrode 100 further comprises an attaching member 130. The attaching member 130 is arranged in contact with the first magnetic material 110 and with the magnet 120 to maintain the distance D between the first magnetic material 110 and the magnet 120. The first magnetic material 110 may further comprise a second surface 114. The second surface 114 may be arranged opposite to the first surface 112. The magnet 120 may comprise a first surface 122.

The first surface 122 of the magnet 120 may face the second surface 114 of the first magnetic material 110. The distance D may correspond to a distance between the first surface 122 of the magnet 120 and the second surface 114 of the first magnetic material 110 along a direction Z extending perpendicular to the first plane XY. The first magnetic material 110 may comprise a second pattern 150 at the first surface 112. The second pattern 150 may comprise a protrusion. Alternatively or in combination, the second pattern 150 may comprise a depression. The first magnetic material 110 may comprise a second pattern 150 at the first surface 112. The second pattern 150 may comprise a protrusion. Alternatively or in combination, the second pattern may comprise a depression. The pattern 150 may comprise any of or any combination of a shape, a line or a dot.

Fig. 2 schematically illustrates a side view of another exemplary electrode 100. The electrode 100 may further comprise a second magnetic material 140. The second magnetic material 140 may extend along the first plane XY. The second magnetic material 140 may comprise a first surface 142. The second magnetic material 140 may comprise a second surface 144. The second surface 144 may be arranged opposite to the first surface 142. The first surface 142 of the second magnetic material 140 may be configured to be arranged on the user's skin. The second surface 144 of the second magnetic material 140 may be configured to magnetically interact with the magnet 120. The second surface 144 of the second magnetic material 140 may be configured to be attached on the first surface 112 of the first magnetic material 110. The second magnetic material 140 may comprise a first pattern 150 at the first surface 142. The first pattern 150 may comprise a protrusion. Alternatively or in combination, the first pattern 150 may comprise a depression. Fig. 2 shows that the second magnetic material 140 comprises a first pattern 150 at the first surface 142. The first pattern 150 may comprise a protrusion. Alternatively or in combination, the first pattern 150 may comprise a depression. The pattern 150 may comprise any of or any combination of a shape, a line or a dot.

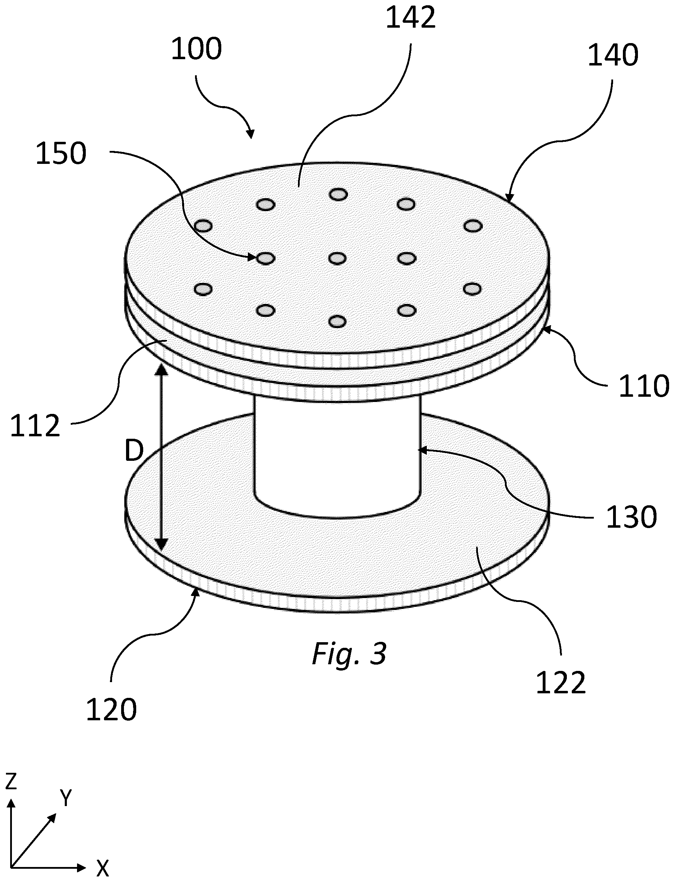

Fig. 3 schematically illustrates a perspective view of yet another exemplary electrode 100. The electrode 100 comprises a first magnetic material 110. The first magnetic material 110 extends along a first plane XY. The first magnetic material 110 comprises a first surface 112. The first surface 112 of the first magnetic material 110 is configured to be arranged on the user's skin. Fig. 3 shows that the electrode 100 further comprises a second magnetic material 140. The second magnetic material 140 may extend along the first plane XY. The second magnetic material 140 may comprise a first surface 142. The electrode 100 further comprises a magnet 120. The magnet 120 may comprise a first surface 122. An attaching member 130 is arranged in contact with the first magnetic material 110 and with the magnet 120 to maintain the distance D between the first magnetic material 110 and the magnet 120.

Fig. 3 shows that the second magnetic material 140 comprises a first pattern 150 at the first surface 142. The first pattern 150 may comprise a protrusion. Alternatively or in combination, the first pattern 150 may comprise a depression. The first magnetic material 110 may comprise a second pattern 150 at the first surface 112. The second pattern 150 may comprise a protrusion. Alternatively or in combination, the second pattern may comprise a depression. The pattern 150 may comprise any of or any combination of a shape, a line or a dot. The first magnetic material 110 may have a round shape such as a circular shape. The second magnetic material 140 may have a round shape such as a circular shape. The magnet 120 may have a round shape such as a circular shape.

The second magnetic material 140 may be the same size as the first magnetic material 110 and/or the magnet 120. The second magnetic material 140 may be smaller than the first magnetic material 110 and/or the magnet 120. The second magnetic material 140 may be larger than the first magnetic material 110 and/or the magnet 120.

The first magnetic material 110 may be the same size as the second magnetic material 140 and/or the magnet 120. The first magnetic material 110 may be smaller than the second magnetic material 140 and/or the magnet 120. The first magnetic material 110 may be larger than the second magnetic material 140 and/or the magnet 120.

The magnet 120 may be the same size as the second magnetic material 140 and/or the first magnetic material 110. The magnet 120 may be smaller than the second magnetic material 140 and/or the first magnetic material 110. The magnet 120 may be larger than the second magnetic material 140 and/or the first magnetic material 110.

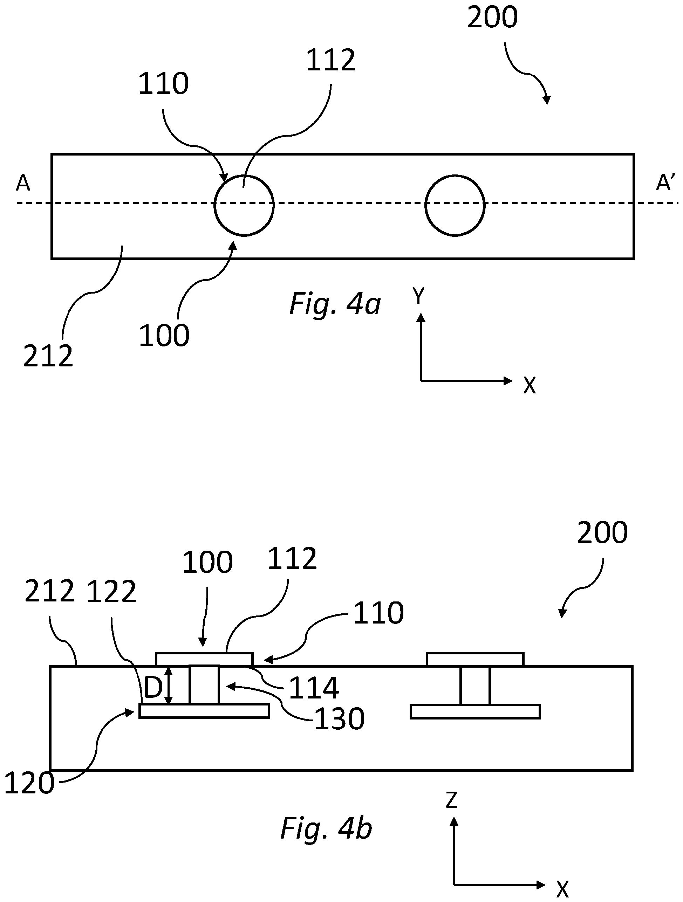

Fig. 4a and 4b schematically illustrate a respective top view and cross-sectional view of an exemplary of wearing device 200. The wearing device 200 is configured to be worn by a user. The wearing device 200 comprises an electrode 100. Fig. 4a shows that the wearing device 200 comprises two electrodes 100. The first magnetic material 110 of the electrode 100 is arranged on a first surface 212 of the wearing device 200. The first surface 212 of the wearing device 200 is configured to be arranged on a user's skin. The magnet 120 and the attaching member 130 of the electrode 100 are arranged in the wearing device 200. The distance D may correspond to a distance between the first surface 122 of the magnet 120 and the second surface 114 of the first magnetic material 110 along a direction Z. the direction Z may extend perpendicular to the first plane XY.

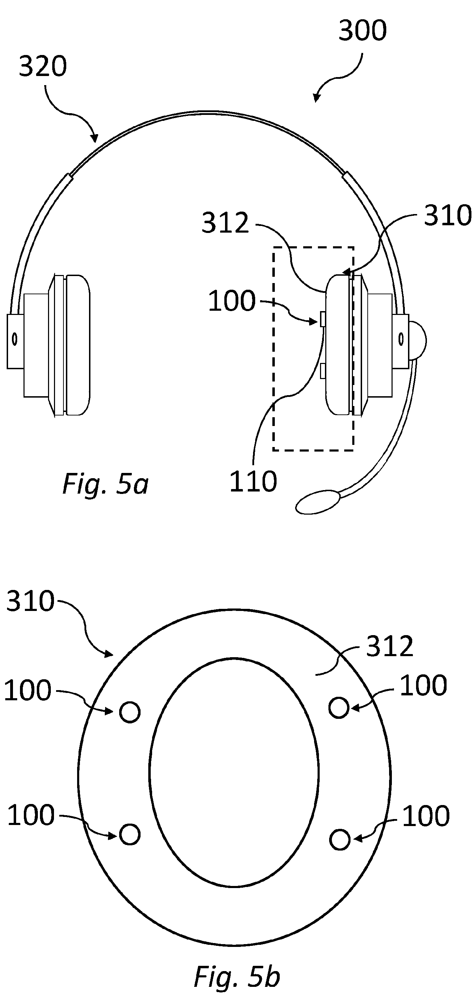

Fig. 5a schematically illustrates a front view of an exemplary a headset 300. The headset 300 may comprise at least an earphone 310 and a headband 320. Fig. 5a shows that the headset comprises two earphones 310. The first magnetic material 110 of the electrode 100 may be arranged on a first surface 312 of the earphone 310. The first surface 312 of the earphone 310 may be configured to be arranged on a user's skin when in use. The magnet 120 and the attaching member 130 of the electrode 100 may be arranged in the earphone 310. The electrodes 100 arranged on the first surface 312 of the earphone 310 may be on the same level as the first surface 312 of the earphone 310. Alternatively, the electrodes 100 may protrude relative to the first surface 312 of the earphone 310, as shown in Fig. 5a. The headset 300 may comprise a plurality of electrodes 100 arranged across the first surface 312 of the earphone 310.

Fig. 5b shows that the headset 300 comprises a plurality of electrodes 100. The plurality of electrodes 100 may be arranged across the first surface 312 of the earphone 310. The plurality of electrodes 100 may be four electrodes 100.

Although particular features have been shown and described, it will be understood that they are not intended to limit the claimed invention, and it will be made obvious to those skilled in the art that various changes and modifications may be made without departing from the scope of the claimed invention. The specification and drawings are, accordingly, to be regarded in an illustrative rather than restrictive sense. The claimed invention is intended to cover all alternatives, modifications and equivalents.

### ITEMS:

1. An electrode (100) configured to be arranged on a user's skin for recording electrical activity, the electrode (100) comprising:
   a first magnetic material (110) extending along a first plane (XY), the first magnetic material (110) comprising a first surface (112) being configured to be arranged on the user's skin,
   a magnet (120) arranged in a distance (D) away from the first magnetic material (110) such that the first magnetic material (110) magnetically interacts with the magnet (120), and
   an attaching member (130) arranged in contact with the first magnetic material (110) and with the magnet (120) to maintain the distance (D) between the first magnetic material (110) and the magnet (120).
2. The electrode (100) according to item 1, wherein the first magnetic material (110) further comprises a second surface (114), arranged opposite to the first surface (112), wherein the magnet (120) comprises a first surface (122) facing the second surface (114) of the first magnetic material (110), and wherein the distance (D) corresponds to a distance between the first surface (122) of the magnet (120) and the second surface (114) of the first magnetic material (110) along a direction (Z) extending perpendicular to the first plane (XY).
3. The electrode (100) according to item 1 or 2, wherein the electrode (100) further comprises a second magnetic material (140) extending along the first plane (XY) and comprising a first surface (142) and a second surface (144), wherein the second surface (144) is arranged opposite to the first surface (142), wherein the first surface (142) of the second magnetic material (140) is configured to be arranged on the user's skin, wherein the second surface (144) of the second magnetic material (140) is configured to magnetically interact with the magnet (120) and be attached on the first surface (112) of the first magnetic material (110).
4. The electrode (100) according to item 3, wherein the second magnetic material (140) comprises a first pattern (150) at the first surface (142), wherein the first pattern (150) comprises a protrusion and/or a depression.
5. The electrode (100) according to any of the preceding items, wherein the first magnetic material (110) comprises a second pattern (150) at the first surface (112), wherein the second pattern (150) comprises a protrusion and/or a depression.
6. The electrode (100) according to item 4 or 5, wherein the first pattern and/or the second pattern (150) comprises any of or any combination of a shape, a line or a dot.
7. The electrode (100) according to any of the items 3-6, wherein the first surface (112) of the first magnetic material (110) comprises an indentation and wherein the second surface (144) of the second magnetic material (140) comprises a corresponding protrusion, wherein when the second surface (144) of the second magnetic material (140) is magnetically attached to the first surface (112) of first magnetic material (110), the protrusion of the second surface (144) of the second magnetic material (140) is received into the indentation of the first surface (112) of the first magnetic material (110).
8. The electrode (100) according to any of the items 3-7, wherein the first magnetic material (110) and/or the second magnetic material (140) comprises a coating layer covering at least the first surface (112, 142) of the respective magnetic material (110, 140).
9. The electrode (100) according to any of the items 3-8, wherein the first magnetic material (110) and/or the second magnetic material (140) has a round shape such as a circular shape.
10. The electrode (100) according to any of the items 3-9, wherein a thickness of the first magnetic material (110) and/or a thickness of the second magnetic material (140) along the direction (Z) is in the range of 0.2 mm to 0.6 mm.
11. The electrode (100) according to any of the items 3-10, wherein a dimension of the first magnetic material (110) and/or a dimension of the second magnetic material (140) along the first plane (XY) is in the range of 5 mm to 20 mm.
12. The electrode (100) according to any of the preceding items, wherein the attaching member (130) comprises a spring.
13. A wearing device (200) configured to be worn by a user, the wearing device (200) comprising an electrode (100) according to any of the preceding items, wherein the first magnetic material (110) of the electrode (100) is arranged on a first surface (212) of the wearing device (200), wherein the first surface (212) of the wearing device (200) is configured to be arranged on a user's skin, wherein the magnet (120) and the attaching member (130) of the electrode (100) are arranged in the wearing device (200).
14. The wearing device (200) according to item 13, wherein the wearing device (200) is a headset (300) comprising at least an earphone (310) and a headband (320), wherein the first magnetic material (110) of the electrode (100) is arranged on a first surface (312) of the earphone (310), wherein the first surface (312) of the earphone (310) is configured to be arranged on a user's skin when in use, wherein the magnet (120) and the attaching member (130) of the electrode (100) are arranged in the earphone (310).
15. The wearing device (200) according to item 14, wherein the headset (300) comprises a plurality of electrodes (100) arranged across the first surface (312) of the earphone (310) such as four electrodes (100).

### LIST OF REFERENCES

- 100: Electrode
- 110: First magnetic material
- 112: First surface of the first magnetic material
- 114: Second surface of the first magnetic material
- 120: Magnet
- 122: First surface of the magnet
- 130: Attaching member
- 140: Second magnetic material
- 142: First surface of the second magnetic material
- 144: Second surface of the second magnetic material
- 150: First pattern/Second pattern
- 200: Wearing device
- 212: First surface of the wearing device
- 300: Headset
- 310: Earphone
- 312: First surface of the earphone
- 320: Headband

## Claims

1. An electrode (100) configured to be arranged on a user's skin for recording electrical activity, the electrode (100) comprising:
a first magnetic material (110) extending along a first plane (XY), the first magnetic material (110) comprising a first surface (112) being configured to be arranged on the user's skin,
a magnet (120) arranged in a distance (D) away from the first magnetic material (110) such that the first magnetic material (110) magnetically interacts with the magnet (120), and
an attaching member (130) arranged in contact with the first magnetic material (110) and with the magnet (120) to maintain the distance (D) between the first magnetic material (110) and the magnet (120).

2. The electrode (100) according to claim 1, wherein the first magnetic material (110) further comprises a second surface (114), arranged opposite to the first surface (112), wherein the magnet (120) comprises a first surface (122) facing the second surface (114) of the first magnetic material (110), and wherein the distance (D) corresponds to a distance between the first surface (122) of the magnet (120) and the second surface (114) of the first magnetic material (110) along a direction (Z) extending perpendicular to the first plane (XY).

3. The electrode (100) according to claim 1 or 2, wherein the electrode (100) further comprises a second magnetic material (140) extending along the first plane (XY) and comprising a first surface (142) and a second surface (144), wherein the second surface (144) is arranged opposite to the first surface (142), wherein the first surface (142) of the second magnetic material (140) is configured to be arranged on the user's skin, wherein the second surface (144) of the second magnetic material (140) is configured to magnetically interact with the magnet (120) and be attached on the first surface (112) of the first magnetic material (110).

4. The electrode (100) according to claim 3, wherein the second magnetic material (140) comprises a first pattern (150) at the first surface (142), wherein the first pattern (150) comprises a protrusion and/or a depression.

5. The electrode (100) according to any of the preceding claims, wherein the first magnetic material (110) comprises a second pattern (150) at the first surface (112), wherein the second pattern (150) comprises a protrusion and/or a depression.

6. The electrode (100) according to claim 4 or 5, wherein the first pattern and/or the second pattern (150) comprises any of or any combination of a shape, a line or a dot.

7. The electrode (100) according to any of the claims 3-6, wherein the first surface (112) of the first magnetic material (110) comprises an indentation and wherein the second surface (144) of the second magnetic material (140) comprises a corresponding protrusion, wherein when the second surface (144) of the second magnetic material (140) is magnetically attached to the first surface (112) of first magnetic material (110), the protrusion of the second surface (144) of the second magnetic material (140) is received into the indentation of the first surface (112) of the first magnetic material (110).

8. The electrode (100) according to any of the claims 3-7, wherein the first magnetic material (110) and/or the second magnetic material (140) comprises a coating layer covering at least the first surface (112, 142) of the respective magnetic material (110, 140).

9. The electrode (100) according to any of the claims 3-8, wherein the first magnetic material (110) and/or the second magnetic material (140) has a round shape such as a circular shape.

10. The electrode (100) according to any of the claims 3-9, wherein a thickness of the first magnetic material (110) and/or a thickness of the second magnetic material (140) along the direction (Z) is in the range of 0.2 mm to 0.6 mm.

11. The electrode (100) according to any of the claims 3-10, wherein a dimension of the first magnetic material (110) and/or a dimension of the second magnetic material (140) along the first plane (XY) is in the range of 5 mm to 20 mm.

12. The electrode (100) according to any of the preceding claims, wherein the attaching member (130) comprises a spring.

13. A wearing device (200) configured to be worn by a user, the wearing device (200) comprising an electrode (100) according to any of the preceding claims, wherein the first magnetic material (110) of the electrode (100) is arranged on a first surface (212) of the wearing device (200), wherein the first surface (212) of the wearing device (200) is configured to be arranged on a user's skin, wherein the magnet (120) and the attaching member (130) of the electrode (100) are arranged in the wearing device (200).

14. The wearing device (200) according to claim 13, wherein the wearing device (200) is a headset (300) comprising at least an earphone (310) and a headband (320), wherein the first magnetic material (110) of the electrode (100) is arranged on a first surface (312) of the earphone (310), wherein the first surface (312) of the earphone (310) is configured to be arranged on a user's skin when in use, wherein the magnet (120) and the attaching member (130) of the electrode (100) are arranged in the earphone (310).

15. The wearing device (200) according to claim 14, wherein the headset (300) comprises a plurality of electrodes (100) arranged across the first surface (312) of the earphone (310) such as four electrodes (100).
